# EUROPEAN PATENT APPLICATION

(11) **EP 4 302 780 A1**
(43) Date of publication of application: **10.01.2024**
(21) Application number: 22763225.4
(22) Date of filing: 01.03.2022
(51) Int. Cl.: A61K 45/00, A23L 33/12, A61K 31/202, A61P 7/04, A61P 9/00, A61P 9/10

(54) **COMPOSITION FOR PREVENTING OR TREATING CEREBROVASCULAR ACCIDENT**

(30) Priority: 03.03.2021 JP 2021033733
(71) Applicant: Tokyo Metropolitan Institute of Medical Science, Tokyo 156-8506 (JP)
(72) Inventor: SHICHITA, Takashi, Tokyo 156-8506 (JP); MURAKAMI, Makoto, Tokyo 113-8654 (JP)
(74) Representative: HGF
(86) International application number: PCT/JP2022/008483
(87) International publication number: WO 2022/186170

(57) **Abstract**

Provided is a composition for preventing or treating cerebrovascular disease in a subject, comprising an activator of peptidylarginine deiminase 4.

## Description

### [Technical Field]

The present invention relates to a composition for preventing or treating cerebrovascular disease, comprising an activator of peptidylarginine deiminase 4.

### [Background Art]

Cerebrovascular disease (stroke) is a leading cause of death among the elderly, and cerebral infarction in particular accounts for about 80% of all stroke cases. Neurological symptoms such as sudden impaired consciousness, brain dysfunction, sensory disturbance, and paralysis of limbs are observed in patients with cerebral infarction, and neurological symptoms worsen within a few days of the onset in about 20 to 30% of patients even with adequate treatment provided in the early phase of the onset. Under current circumstances, however, there is no effective treatment for cerebral infarction, except for reperfusion therapy in the hyperacute phase within a few hours of the onset of cerebral infarction, and the only approach for restoring neurological function is rehabilitation. Therefore, it is urgently needed to develop a therapeutic agent that can restore the cerebral tissue damaged by cerebral infarction and improve prognosis.

One of the problems in developing a therapeutic agent for cerebral infarction is that the brain has a blood-brain barrier that makes it difficult for therapeutic agents to cross over into the cerebral tissue. In this regard, lipids are small molecules that migrate well into cerebral tissue, and therefore, lipids with cerebral tissue protecting or repairing activity are likely to be promising as therapeutic agents for cerebral infarction. It has been known that while ω-6 fatty acids such as prostaglandin and leukotriene or their metabolites promote inflammation, ω-3 fatty acids such as docosahexaenoic acid and eicosapentaenoic acid or their metabolites inhibit inflammation. However, dynamics of these lipids in cerebrovascular disease have not been clarified. For example, it has been revealed that nonsteroidal anti-inflammatory agents that inhibit production of inflammatory lipid mediators such as prostaglandin do not improve prognosis in patients with stroke (Non-Patent Document 1).

### [Citation List]

### [Non-Patent Document]

[Non-Patent Document 1] Iadecola C., Anrather J., 2011, Nat Med., 17:796-808

### [Summary of Invention]

### [Technical Problem]

The present invention was made to provide a therapeutic agent for cerebral infarction that is effective after the onset of stroke, especially in the subacute phase 24 hours or more after the onset.

### [Solution to Problem]

As a result of diligent research, the inventors found that a dihomo-γ-linolenic acid metabolite can improve neurological symptoms after cerebral infarction by activating peptidylarginine deiminase 4.

That is, according to one embodiment, the present invention provides a composition for preventing or treating cerebrovascular disease in a subject, comprising an activator of peptidylarginine deiminase 4.

The activator of peptidylarginine deiminase 4 is preferably a dihomo-γ-linolenic acid metabolite, and 15-hydroxyeicosatrienoic acid or a metabolite thereof is especially preferred.

The cerebrovascular disease is preferably selected from the group consisting of cerebral hemorrhage, subarachnoid hemorrhage, cerebral infarction, and cerebrovascular dementia.

The composition is preferably a pharmaceutical agent, and it is further preferred that it be administered within 48 hours of the onset of cerebrovascular disease.

Alternatively, the composition is preferably a food or a drink.

According to another embodiment, the present invention provides a method of preventing or treating cerebrovascular disease in a subject, comprising a step of administering to the subject an activator of peptidylarginine deiminase 4, such as a dihomo-γ-linolenic acid metabolite.

According to yet another embodiment, the present invention also provides a use of an activator of peptidylarginine deiminase 4, such as a dihomo-γ-linolenic acid metabolite, in prevention or treatment of cerebrovascular disease in a subject, or in a pharmaceutical agent or a food or a drink therefor.

### [Advantageous Effects of Invention]

The composition for preventing or treating cerebrovascular disease of the present invention can not only prevent cerebrovascular disease, but also improve neurological symptoms after onset, especially even in the subacute phase 24 hours or more after onset.

### [Brief Description of Drawings]

[Fig. 1] Fig. 1 is a heat map showing the content of lipid metabolites in the infarcted cerebral tissue of a wild-type mouse model.
[Fig. 2] Fig. 2 is a graph showing the level of PLA2g2e expression in the infarcted cerebral tissue of a wild-type mouse model.
[Fig. 3] Fig. 3 is a chart showing changes over time in neurological symptoms in a cerebral infarction model of wild-type mice and in a cerebral infarction model of PLA2g2e knockout mice.
[Fig. 4] Fig. 4 shows immunohistochemistry images of infarcted cerebral tissues of a wild-type mouse model and a PLA2g2e knockout mouse model using anti-MAP2 antibody.
[Fig. 5] Fig. 5 is a heat map showing gene expression patterns in the infarcted cerebral tissue of wild-type mice and PLA2g2e knockout mice.
[Fig. 6] Fig. 6 shows an immunofluorescence staining image of the infarcted cerebral tissue of a wild-type mouse model using anti-PADI4 antibody.
[Fig. 7] Fig. 7 is a graph showing the content of dihomo-γ-linolenic acid in the infarcted cerebral tissue of a wild-type mouse model and a PLA2g2e knockout mouse model.
[Fig. 8] Fig. 8 is a chart showing changes over time in the neurological symptoms of cerebral infarction models of wild-type mice treated or not treated with 15-hydroxyeicosatrienoic acid.
[Fig. 9] Fig. 9 is a chart showing Padi4 promoter inducing activity of various lipids.
[Fig. 10] Fig. 10 is a chart comparing cerebral infarct volumes in cerebral infarction models of PLA2g2e knockout mice treated with DGLA, stearic acid or vehicle and wild-type mice.
[Fig. 11] Fig. 11 is a chart showing changes over time in neurological symptoms in cerebral infarction models of PLA2g2e knockout mice treated with DGLA, stearic acid, or vehicle and wild-type mice.
[Fig. 12] Fig. 12 is a chart comparing the number of Padi4-expressing cells around the cerebral infarct area in PLA2g2e knockout mouse models treated with DGLA, stearic acid or vehicle and a wild-type mouse model.
[Fig. 13] Fig. 13 is a chart showing changes over time in neurological symptoms in cerebral infarction models of Padi4 floxed mice, neuron-specific Padi4 cKO mice, and myeloid cell-specific Padi4 cKO mice.
[Fig. 14] Fig. 14 is a UMAP plot showing the results of single cell RNA-seq analysis of neurons around the infarct area isolated from cerebral infarction models of neuron-specific Padi4 cKO mice and wild-type mice.
[Fig. 15] Fig. 15 is a chart comparing the cerebral infarct volume in 15-HETrE-treated and vehicle-treated groups of a wild-type mouse cerebral infarction model.
[Fig. 16] Fig. 16 is a chart showing the results of a corner test for 15-HETrE-treated and vehicle-treated groups of a wild-type mouse cerebral infarction model.
[Fig. 17] Fig. 17 is a chart showing the results of a cylinder test for 15-HETrE-treated and vehicle-treated groups of wild-type mouse cerebral infarction model.
[Fig. 18] Fig. 18 is a volcano plot showing the results of RNA-seq analysis of neurons around the infarct area isolated from 15-HETrE-treated and vehicle-treated groups of a wild-type mouse cerebral infarction model.

### [Description of Embodiments]

The present invention is described in detail below, but the invention is not limited to the embodiments described herein.

According to a first embodiment, the present invention is a composition for preventing or treating cerebrovascular disease in a subject, comprising an activator of peptidylarginine deiminase 4.

According to the embodiment, "preventing" includes, not only preventing the onset of cerebrovascular disease in a subject who may develop cerebrovascular disease, but also reducing the risk of developing cerebrovascular disease, and by providing a procedure before the subject develops cerebrovascular disease, delaying the progression or reducing the severity of symptoms if the subject develops cerebrovascular disease.

According to the embodiment, "treating" includes, not only completely curing cerebrovascular disease, but also causing remission or alleviation of symptoms of cerebrovascular disease, delaying or stopping the progression of cerebrovascular disease, and improving prognosis of cerebrovascular disease.

According to the embodiment, "cerebrovascular disease" means hemorrhagic cerebrovascular disease, ischemic cerebrovascular disease and all brain dysfunctions resulting therefrom. Thus, cerebrovascular disease according to the embodiment includes, but is not limited to, cerebral hemorrhage; subarachnoid hemorrhage; cerebral infarction such as lacunar infarction, atherothrombotic cerebral infarction, and cardiogenic cerebral embolism; and neurocognitive disorders resulting from vascular disorder or neurodegeneration, such as cerebrovascular dementia. Cerebrovascular disease according to the embodiment is preferably selected from the group consisting of cerebral hemorrhage, subarachnoid hemorrhage, cerebral infarction, and cerebrovascular dementia.

The "subject" according to the embodiment may be any vertebrate, but is preferably a mammal such as a mouse, a rat, a rabbit, a sheep, a pig, a cow, a goat, a monkey, a human, and especially preferably a human. The subject may be of any age, including an infant, a youth, an adolescent, an adult, and an elderly animal.

The "activator of peptidylarginine deiminase 4 (PADI4)" according to the embodiment may be any substance that can directly or indirectly increase expression and/or activity of PADI4. Thus, the activator of PADI4 that may be used according to the embodiment includes, but is not limited to, a protein, a peptide, a nucleic acid, a lipid, and a low molecular weight compound. In addition, such a substance may be a publicly known one or a novel one.

The activator of PADI4 according to the embodiment is preferably a lipid, and more preferably a dihomo-γ-linolenic acid metabolite. The "dihomo-γ-linolenic acid (DGLA) metabolite" that may be used as the activator of PADI4 according to the embodiment includes, but is not limited to, 15-hydroxyeicosatrienoic acid (15-HETrE), 12-hydroxyeicosatrienoic acid (12-HETrE), 8-hydroxyeicosatrienoic acid (8-HETrE), prostaglandin E1, prostaglandin F1 and metabolites thereof. The activator of PADI4 that may be used according to the embodiment is preferably 15-HETrE or a metabolite thereof.

The composition of the embodiment may comprise one or more activators of PADI4 selected from the above as one or more active ingredients. In the composition of the embodiment, the activator of PADI4 should be included in an appropriate amount to be ingested by the subject. For example, in the case of using 15-HETrE as the activator of PADI4, 15-HETrE may be included in the composition in an amount such that the intake is, for example, 0.01 to 1 mg/kg (body weight), preferably 0.03 to 0.1 mg/kg (body weight), per day for an adult. However, the amount is not limited to such a range and may be adjusted according to the form of the composition, conditions, age or gender of the subject, and the like.

The composition of the embodiment may be composed of the active ingredient only, but generally, may also further comprise pharmaceutically acceptable known carriers and additives as optional ingredients.

The composition of the embodiment may be produced, for example, as a pharmaceutical agent. In this case, the composition may be formulated into various dosage forms, such as tablets, granules, powders, capsules, jellies, syrups, and injectable compositions. Accordingly, the composition of the embodiment may be administered by various methods, including oral, intraperitoneal, intravenous, subarachnoid, and intracerebral administrations. The composition of the embodiment is preferably prepared as an oral formulation or an enteral nutrient, and it is therefore preferably administered orally or enterally.

If the pharmaceutical agent of the embodiment is prepared as an oral formulation, it may be a solid formulation such as tablets, granules, and powders. In this case, appropriate additives, such as starch, mannitol, carboxymethylcellulose, cornstarch, and inorganic salts, and if desired, binders, disintegrators, and lubricants, may be blended. If the pharmaceutical agent of the embodiment is prepared as tablets, the composition may be coated with sucrose, gelatin, hydroxypropyl cellulose, and the like, if desired. If the pharmaceutical agent of the embodiment is prepared as a liquid formulation such as syrups, sterile water, saline solutions, ethanol, and the like may be used as a carrier, and if desired, adjuvants such as suspending agents may be added.

If the pharmaceutical agent of the embodiment is prepared as a parenteral formulation, it may be a liquid formulation such as injectable composition. In this case, it may be prepared by dissolving or suspending the active ingredient in diluents such as distilled water for injection, saline solutions, aqueous solutions of glucose, vegetable oils for injection, or polyethylene glycol, and adding disinfectants, tonicity agents, or soothing agents, as appropriate.

The pharmaceutical agent of the embodiment may further be blended with pharmaceutically acceptable additives, such as preservatives and stabilizers, and other therapeutic agents, if desired.

Alternatively, the composition of the embodiment may be produced as a food or a drink. In this case, the composition of the embodiment may be, for example, a processed food for general use. The processed food for general use includes, but is not limited to, breads, noodles, confectionery, edible oils and fats, seasonings, and beverages.

In addition, the composition of the embodiment may be a nutritional supplementary food. The nutritional supplementary food may include, for example, supplements, nutritional beverages, food for specified health uses, food with nutrient function claims, and the like. The nutritional supplementary food of the embodiment may be prepared, for example, as powders, granules, tablets, capsules, jellies, or liquid formulations, and may be further blended with additives such as flavorings, colorants, sweeteners and preservatives, and other nutritional ingredients, if desired.

The composition of the embodiment may be administered to or ingested by the subject before or after the onset of cerebrovascular disease. If administered after the onset of cerebrovascular disease, the composition may be administered to the subject within 48 hours of the onset of cerebrovascular disease, and is preferably administered to the subject 24 hours or more after, and within 48 hours of the onset of, cerebrovascular disease.

The composition of the embodiment is useful for preventing or treating cardiovascular disease. In particular, since the composition of the embodiment can exert its therapeutic effect even when administered 24 hours or more after the onset of cerebrovascular disease, it is useful for the treatment in the subacute phase of cerebrovascular disease. In addition, the composition of the embodiment is useful for preventing neurocognitive disorders resulting from vascular disorder and neurodegeneration, such as cerebrovascular dementia.

According to a second embodiment, the present invention is a method of preventing or treating cerebrovascular disease in a subject, comprising a step of administering to the subject an activator of peptidylarginine deiminase 4. The terms "preventing," "treating," "cerebrovascular disease," "subject," and "activator of peptidylarginine deiminase 4" used in this embodiment are the same as those defined in the first embodiment.

In the method of the embodiment, the activator of peptidylarginine deiminase 4 may be formulated into various dosage forms as defined in the first embodiment, and may be administered by any method of administration suitable for the dosage form, such as oral, intraperitoneal, intravenous, subarachnoid, and intracerebral administrations. The dosage of the activator of peptidylarginine deiminase 4 should be in a range appropriate to the dosage form and should be such that the intake is as defined in the first embodiment.

According to the method of the embodiment, the activator of peptidylarginine deiminase 4 may be administered to the subject before or after the onset of cerebrovascular disease in the subject, and it is preferably administered to the subject who is in the subacute phase of cerebrovascular disease (i.e., within 48 hours of the onset of cerebrovascular disease, especially 24 hours or more after and within 48 hours of the onset).

According to a third embodiment, the present invention is a use of an activator of peptidylarginine deiminase 4, such as a dihomo-γ-linolenic acid metabolite, in prevention or treatment of cerebrovascular disease, or in a pharmaceutical agent or a food or a drink therefor. The terms "preventing," "treating," "cerebrovascular disease," and "activator of peptidylarginine deiminase 4" used in this embodiment are the same as those defined in the first embodiment. The activator of peptidylarginine deiminase 4 may be used for preventing or treating cerebrovascular disease or for producing a pharmaceutical agent or a food or a drink therefor, according to the description in the first and the second embodiments.

### [Examples]

The present invention is further explained by giving Examples below. These are not intended to limit the invention at all.

### 1. Comprehensive exploration of lipids associated with protection and repair of infarcted cerebral tissue

To identify lipids associated with pathological conditions of cerebral infarction, a comprehensive analysis was performed on lipids extracted from the cerebral tissue of a mouse model of cerebral infarction. The mouse model of cerebral infarction was prepared according to the following procedure. An embolization suture with a silicone-coated tip (Ethilon (suture gauge number 7-0), Johnson & Johnson) was inserted through the common carotid artery of 8 to 24-week-old C57BL/6N mice. Cerebral temperature was maintained at 36°C, cerebral blood flow was monitored by Doppler blood flowmeter ALF21 (ADVANCE CO., LTD.), and the embolization suture was placed at the origin of the middle cerebral artery so that the cerebral blood flow in the perfused area of the middle cerebral artery was maintained at 20 to 40% of the steady state. By removing the embolization suture after 60 minutes of ischemia, the cerebral tissue was reperfused. As a control, sham-operated mice were prepared.

The cerebral tissues were isolated from the mouse model of cerebral infarction and sham-operated mice 1, 3, and 6 days after ischemia-reperfusion. Each of cerebral tissues was homogenized in methanol and allowed to stand overnight to obtain a lipid extract. By adding ultrapure water, the lipid extract was made into a 10% methanol solution and was submitted to a solid phase extraction cartridge Oasis HLB 3cc Vac (Waters) after adjusting pH to 3 to 4 using hydrochloric acid. Lipids were eluted by methyl formate, evaporated to dryness by nitrogen gas, and then dissolved in an aqueous 60% methanol solution. The lipid solution obtained was subjected to high performance liquid chromatography-mass spectrometry (HPLC/MS/MS) (4000Q TRAP triple quadrupole mass spectrometer (AB Sciex), electrospray ionization positive mode, multiple reaction monitoring) for a comprehensive quantitative analysis of lipids in the solution. Deuterium-labeled eicosapentaenoic acid was used as an endogenous control.

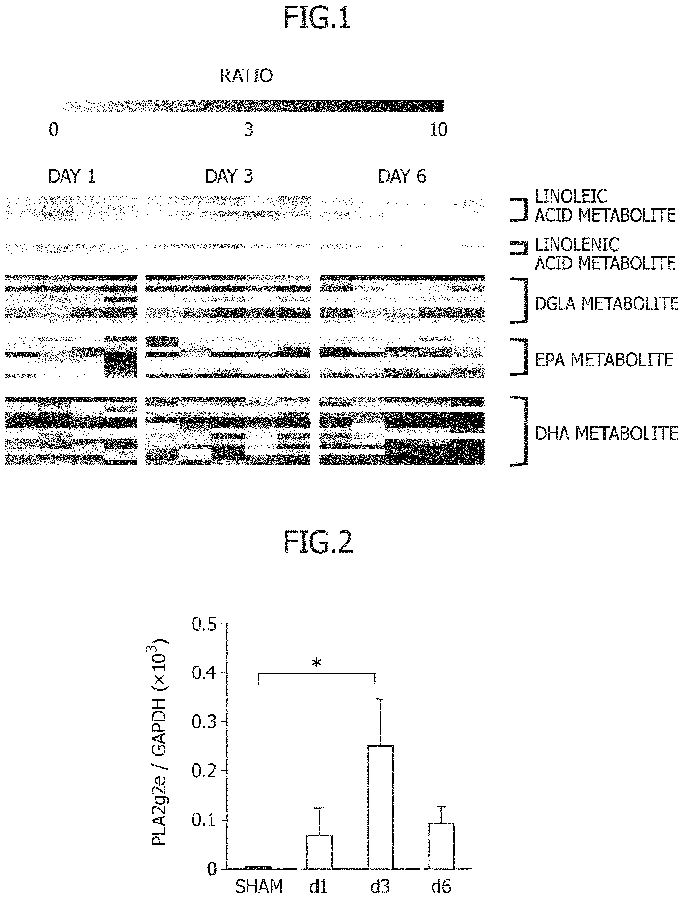

The results are shown in Fig. 1. In the figure, "Ratio" indicates the ratio of the lipid content in the cerebral tissue derived from the mouse model of cerebral infarction (infarcted cerebral tissue) to the lipid content in the cerebral tissue derived from sham-operated mice (normal cerebral tissue). It was found that metabolites of eicosapentaenoic acid (EPA) and docosahexaenoic acid (DHA), which are known bioactive lipids, increased in the infarcted cerebral tissue. Furthermore, it was discovered that metabolites of dihomo-γ-linolenic acid (DGLA) metabolites increased in the infarcted cerebral tissue. These results suggest that metabolites of polyunsaturated fatty acids may regulate pathological conditions of cerebral infarction.

### 2. Identification of PLA2 subtypes associated with protection and repair of infarcted cerebral tissue

Polyunsaturated fatty acids are released from the cell membrane into the cell by phospholipase A2 (PLA2) and are metabolized by fatty acid metabolizing enzymes in the cell. Thus, the expression of PLA2 subtypes in the normal cerebral tissue and the infarcted cerebral tissue was analyzed by microarray and quantitative PCR. A mouse model of cerebral infarction and sham-operated mice were prepared, and the cerebral tissues were isolated after perfusion with a physiological saline solution by the same procedure as in 1 above. RNA was extracted from the cerebral tissues using RNAmicro (Qiagen) and the microarray analysis was performed. The results confirmed that PLA2g2e was strongly expressed in the infarcted cerebral tissue, whereas it was not expressed in the normal cerebral tissue (data not shown).

Next, the expression of PLA2g2e in the normal cerebral tissue and the infarcted cerebral tissue was analyzed by quantitative PCR. Mouse models of cerebral infarction and sham-operated mice were prepared (n=4, respectively) and the cerebral tissues were isolated after perfusion with a physiological saline solution by the same procedure as in 1 above. RNA was extracted from 1 mm³ of the cerebral tissue by RNAiso Plus (Takara Bio Inc.) and cDNA was prepared using High-Capacity cDNA Reverse Transcription Kit (Thermo Fisher Scientific). The obtained cDNA was used as a template and quantitative PCR was performed on a CFX96 Real-Time System device (Bio-Rad Laboratories) using SsoFast EvaGreen Supermix (Bio-Rad Laboratories). GAPDH was used as an internal standard gene and the level of PLA2g2e expression was normalized. The primer sets used for the quantitative PCR are shown below.

### Table 1. Primer set for quantitative PCR

**[Table 1]**

| Gene | Forward/reverse primer (5'→3') | SEQ ID No: |
|---|---|---|
| *Pla2g2e* | GACCCCAAGCTGGAAAAGTA | 1 |
| | TAGTGGGCATACTTGCGGTT | 2 |
| *Gapdh* | AACTTTGGCATTGTGGAAGG | 3 |
| | GGATGCAGGGATGATGTTCT | 4 |

The results are shown in Fig. 2. In the figure, "Sham" indicates sham-operated mice (control), "d1" indicates mouse models of cerebral infarction 1 day after ischemia-reperfusion, "d3" indicates mouse models of cerebral infarction 3 days after ischemia-reperfusion, and "d6" indicates mouse models of cerebral infarction 6 days after ischemia-reperfusion. It was confirmed that PLA2g2e expression in the infarcted cerebral tissue increased over time up to 3 days after ischemia-reperfusion. (Fig. 2, *: p < 0.05 vs. control group (analysis by Dunnett's multiple comparison test)).

Then, cerebral infarction models of PLA2g2e knockout mice (Sato H., et al., 2014, Cell Metab, 20: 119-132) and wild-type mice were prepared by the same procedure as in 1 above (n = 12, respectively), and neurological symptoms after ischemia-reperfusion were assessed over time in accordance with the neurological symptoms assessment score reported by Bederson, et al. (Bederson JB, et al., 1986, Stroke, 17: 472-476). Scoring criteria are as follows: grade 0, no neurological symptoms observed; grade 1, some flexion of forelimb observed; grade 2, no resistance felt when pushing the mouse from the lateral toward the paretic side; grade 3, the mouse moves circling toward the paretic side.

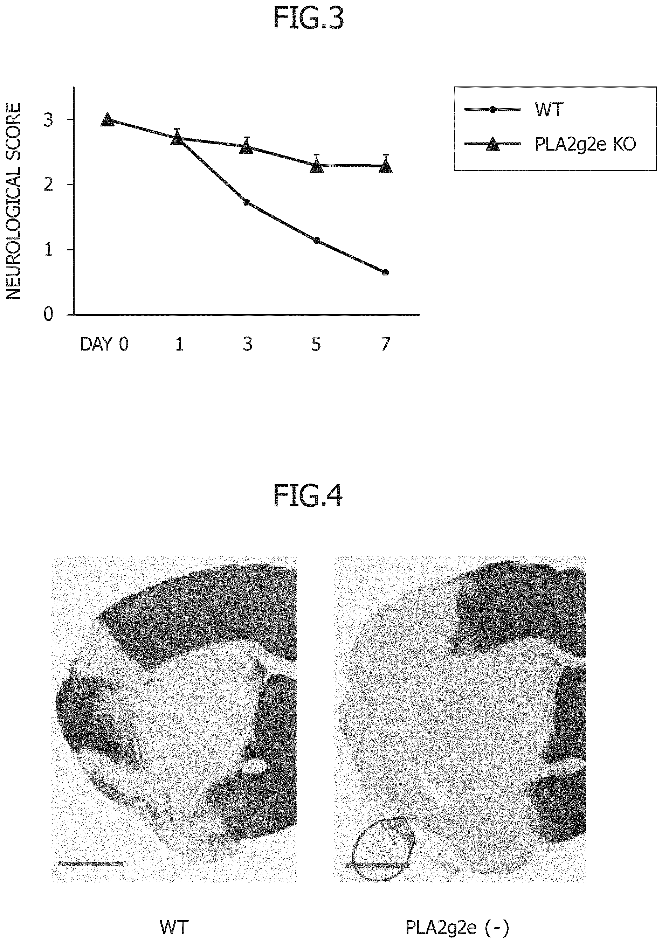

The results are shown in Fig. 3. The cerebral infarction models of PLA2g2e knockout mice exhibited significantly worse neurological symptoms compared to the cerebral infarction models of wild-type mice.

Then, mice were perfused and fixed transcardially using a 4% paraformaldehyde solution 7 days after ischemia-reperfusion, and the brains were isolated. From the isolated cerebral tissue, sections of 1 mm thickness were prepared, embedded in paraffin, thinly sliced to 5 µm, and immunohistochemistry was performed according to an ordinary method. Anti-MAP2 antibody (Sigma-Aldrich, Cat. #M4403) (1:1000 dilution) was used as a primary antibody, and anti-mouse IgG antibody derived from goat (Jackson Immuno Research Laboratories, Inc., Cat. # 115-005-003) (1:300 dilution) was used as a secondary antibody.

The results are shown in Fig. 4. In the figure, the scale bar indicates 1 mm, and the round linear object is an air bubble that was mixed in during the preparation of the specimen. The ischemic necrotic region (unstained region) in the cerebral infarction model of PLA2g2e knockout mice has expanded compared to the cerebral infarction model of wild-type mice. Also, immunohistochemistry was performed for confirming PLA2g2e expression in wild-type mice, and it was confirmed that while no PLA2g2e expression was observed in the normal cerebral tissue, the expression with a peak on day 3 from the onset was observed in the infarcted cerebral tissue (data not shown). These results indicate that PLA2g2e is a phospholipid-metabolizing enzyme associated with protection and repair of the cerebral tissue in the subacute phase of several days after the onset of cerebral infarction.

### 3. Comprehensive exploration of genes associated with protection and repair of infarcted cerebral tissues

Cerebral infarction models of PLA2g2e knockout mice and wild-type mice were prepared by the same procedure as in 1 above. The cerebral tissue was isolated on day 3 of the onset, and RNA was extracted from the cerebral tissues using RNeasy Mini Kit (Qiagen). The quality of the RNA was confirmed using 2100 Bioanalyzer (Agilent Technologies), and cDNA was synthesized using Low Input QuickAmp Labeling Kit (Agilent Technologies). The resulting cDNA was hybridized using Whole Mouse Genome DNA Microarray Kit 4×44K (Agilent Technologies), which was washed, and then data was acquired using SureScan Microarray Scanner (Agilent Technologies). The obtained data were analyzed by GeneSpring GX software.

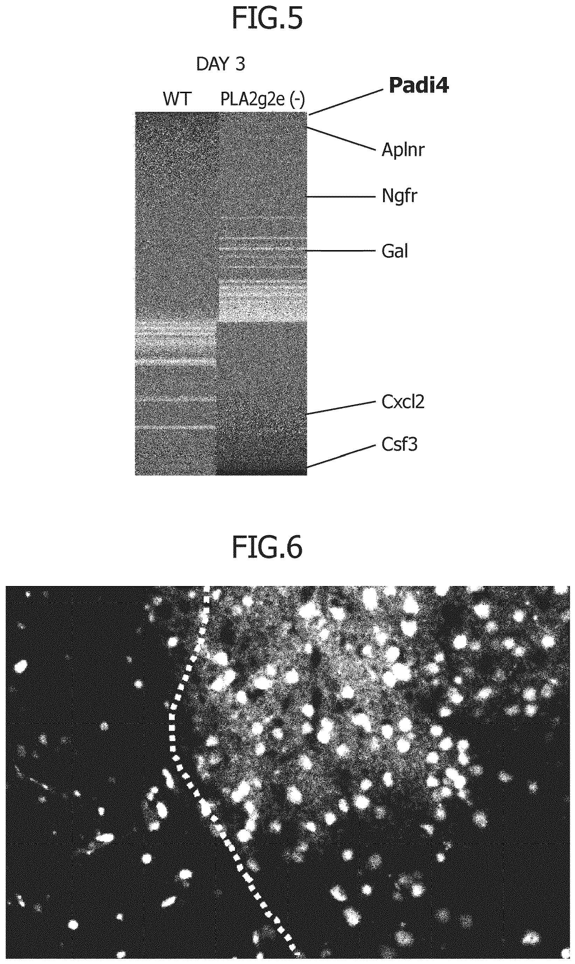

The results are shown in Fig. 5. It was confirmed that while the expression of inflammatory cytokine genes such as chemokine CXCL2 and colony-stimulating factor 3 (Csf3) markedly increased in the cerebral infarction model of PLA2g2e knockout mice compared to the cerebral infarction model of wild-type mice, the expression of genes associated with neuronal repair, such as apelin receptor (Aplnr), low-affinity nerve growth factor receptor (Ngfr), and galanin (Gal), decreased; in particular, the expression of peptidylarginine deiminase 4 (Padi4) gene significantly decreased.

Cerebral tissue slices were also prepared from wild-type mice 3 days after ischemia-reperfusion by the same procedure as in 2 above, and fluorescent double staining was performed using anti-PADI4 antibody (rabbit polyclonal, GeneTex, Cat. #GTX113945) (1: 100 dilution) and anti-NeuN antibody (mouse monoclonal, Merck Millipore, Cat. #MAB377) (1:1000 dilution) as the primary antibody, and Alexa488-labeled secondary antibody (anti-rabbit IgG, Thermo Fisher Scientific, Cat. #A11070) (1:500 dilution) and Alexa546-labeled secondary antibody (anti-mouse IgG, Thermo Fisher Scientific, Cat. #A11018) (1:500 dilution) as the secondary antibody.

The results are shown in Fig. 6. In the figure, the left side of the dotted line indicates the cerebral infarct region. A strong PADI4 expression was observed in a region adjacent to the cerebral infarct region (upper right). The inventors also generated neuron-specific PADI4-deficient mice and prepared a cerebral infarction model, and it was observed that neurological symptoms worsened and the cerebral infarct volume increased in the cerebral infarction model of neuron-specific PADI4-deficient mice (data not shown). Although physiological functions of PADI4 in neurons have not been reported, it was known that PADI4 is an enzyme that converts arginine residues in protein to citrulline residues and is involved in the regulation of gene expression by citrullinating histones (Christophorou, MA, et al. 2014, Nature, 507: 104-108). These results suggest that increasing PADI4 expression and/or activity in neurons may protect or repair the infarcted cerebral tissue.

### 4. Identification of PADI4 expression inducing lipids

Cerebral infarction models of PLA2g2e knockout mice and wild-type mice were prepared by the same procedure as in 1 above (n = 6, respectively), and dihomo-γ-linolenic acid (DGLA) was quantified by the same method as in 1 above.

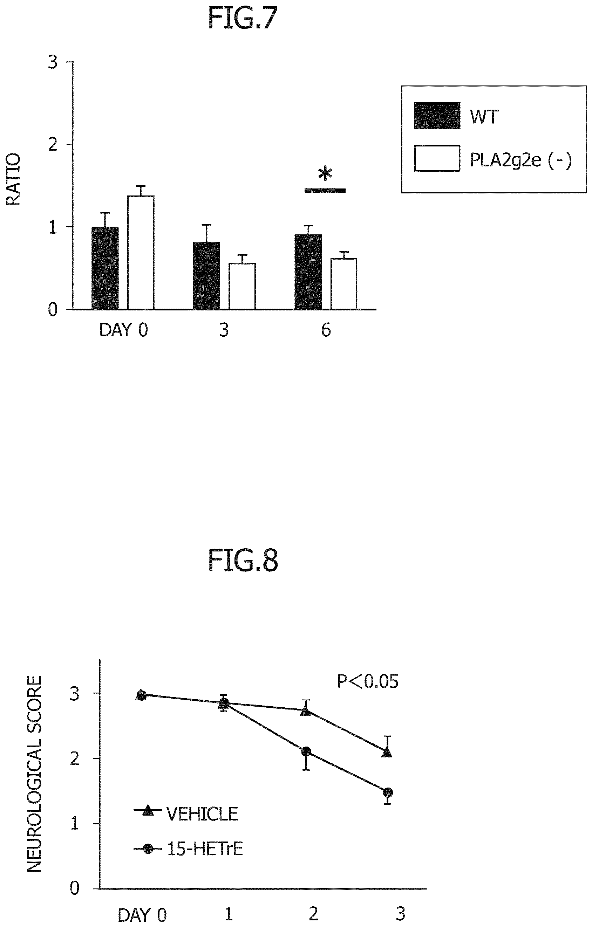

The results are shown in Fig. 7. It was confirmed that the cerebral infarction model of PLA2g2e knockout mice showed decreased DGLA content compared to the cerebral infarction model of wild-type mice (Fig. 7, *: p < 0.05 vs. wild-type mice (Student's t-test)).

Next, a test was conducted to see whether 15-hydroxyeicosatrienoic acid (15-HETrE), a DGLA metabolite, could protect or repair the infarcted cerebral tissue. Cerebral infarction models were prepared using wild-type mice by the same procedure as in 1 above. 24 hours after ischemia-reperfusion, 600 µg of 15-HETrE (Cayman Chemical Company) per mouse (20 to 25 g body weight) was administered intraperitoneally (n = 10, respectively). Neurological symptoms over time after ischemia-reperfusion were evaluated according to the same procedure as in 2 above. Controls were treated with phosphate buffer containing 10% ethanol (vehicle).

The results are shown in Fig. 8. It was confirmed that neurological symptoms were improved by administering 15-HETrE. It was known that DGLA and its metabolites migrate well into the cerebral tissue and are accumulated in the cerebral tissue even by oral intake (Umeda-Sawada R, et al. 2006, Biosci Biotechnol Biochem, 70: 2121-2130), and 15-HETrE was considered promising as a therapeutic agent for cerebral infarction.

### 5. Identification of PADI4 expression inducing lipids (2)

To identify the lipids inducing PADI4 expression, a luciferase reporter vector was prepared by incorporating the promoter region (SEQ ID NO: 5) of the mouse Padi4 gene (NCBI database accession number: NM_011061) into pGL3-Basic (Promega). The reporter vector was transfected into Neuro2A cells using PEI Max (Polysciences), and reporter cells were obtained. After reporter cells were cultured in media supplemented with lipids (10 µM final concentration) for 24 hours, the luciferase activity was measured with Luciferase Assay System (Promega). The lipids used are as follows: docosahexaenoic acid (DHA) (Sigma-Aldrich) and as a DHA metabolite, tetracosahexaenoic acid (THA) (Cayman Chemical Company); eicosapentaenoic acid (EPA) (Sigma-Aldrich) and as an EPA metabolite, methyl all-cis-7,10,13,16,19-docosapentaenoate (DPA) (n-3) (Cayman Chemical Company); DGLA (Cayman Chemical Company) and as DGLA metabolites 8(S)-HETrE (Cayman Chemical Company), 12(S)-HETrE (Cayman Chemical Company), 15(S)-HETrE (Cayman Chemical Company), prostaglandin E1 (Cayman Chemical Company), 15-keto prostaglandin E1 (Cayman Chemical Company) and prostaglandin F1α (Cayman Chemical Company); arachidonic acid (AA) (Sigma-Aldrich) and as AA metabolites 5(S)-HETE (Cayman Chemical Company), 8(S)-HETE (Cayman Chemical Company), 12(S)-HETE (Cayman Chemical Company), 15(S)-HETE (Cayman Chemical Company), prostaglandin E2 (Cayman Chemical Company), prostaglandin F2α (Cayman Chemical Company) and 13,14-dihydro-15-keto prostaglandin E2 (Cayman Chemical Company); and as other fatty acid metabolites, palmitic acid (Sigma-Aldrich), stearic acid (Sigma-Aldrich), α-linolenic acid (Sigma-Aldrich), 9(S)-HpOTrE (Cayman Chemical Company), 13(S)-HpOTrE (Cayman Chemical Company), 9(S)-HOTrE (Cayman Chemical Company), 13(S)-HOTrE (Cayman Chemical Company), methyl 11,14,17-icosatrienoate (Cayman Chemical Company), stearidonic acid (Sigma-Aldrich), linoleic acid (Sigma-Aldrich) and 13(S)-HODE (Cayman Chemical Company).

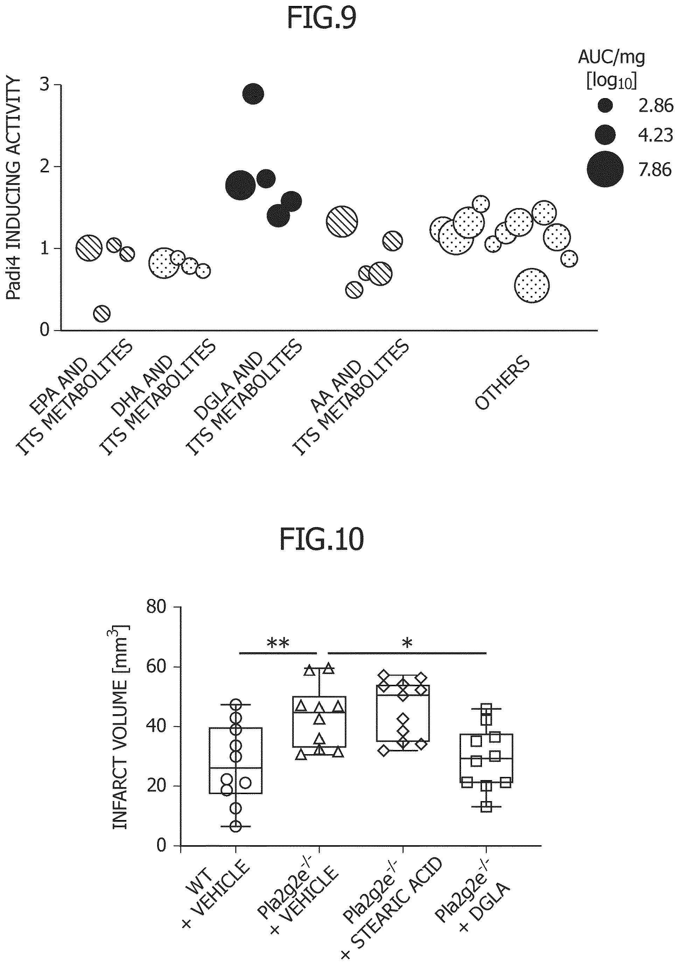

The results are shown in Fig. 9. The vertical axis of the graph indicates Padi4 promoter inducing activity based on the luciferase activity (relative value with the Padi4 promoter inducing activity in untreated cells set as 1). The size of the circles indicates the content of each lipid in the cerebral tissue of the cerebral infarction model of wild-type mice. It was confirmed that only DGLA and its metabolites induce Padi4 promoter activity.

### 6. Identification of PADI4 expression inducing lipids (3)

Cerebral infarction models of PLA2g2e knockout mice and wild-type mice were prepared by the same procedure as in 1 above, and DGLA (1 mg/ethanol) was orally administered using an oral probe for mice 24, 48, and 72 hours after ischemia-reperfusion. To controls, vehicle (ethanol) or stearic acid (1 mg/ethanol) was orally administered instead of DGLA (n = 10, respectively).

Sections were prepared from the cerebral tissue 4 days after ischemia-reperfusion and immunohistochemistry was performed using anti-MAP2 antibody by the same procedure as in 2 above. The cerebral infarct volume was calculated based on the region that was not stained by the anti-MAP2 antibody, which is regarded as the cerebral infarct area. The results are shown in Fig. 10. The cerebral infarct volume of the cerebral infarction model of the PLA2g2e knockout mice markedly increased compared to the cerebral infarction model of wild-type mice (** p < 0.01, two-way ANOVA with Dunnett's post-test). However, administration of DGLA to the cerebral infarction model of PLA2g2e knockout mice decreased the cerebral infarct volume (* p < 0.05, two-way ANOVA with Dunnett's post-test). In contrast, administration of stearic acid did not change the cerebral infarct volume.

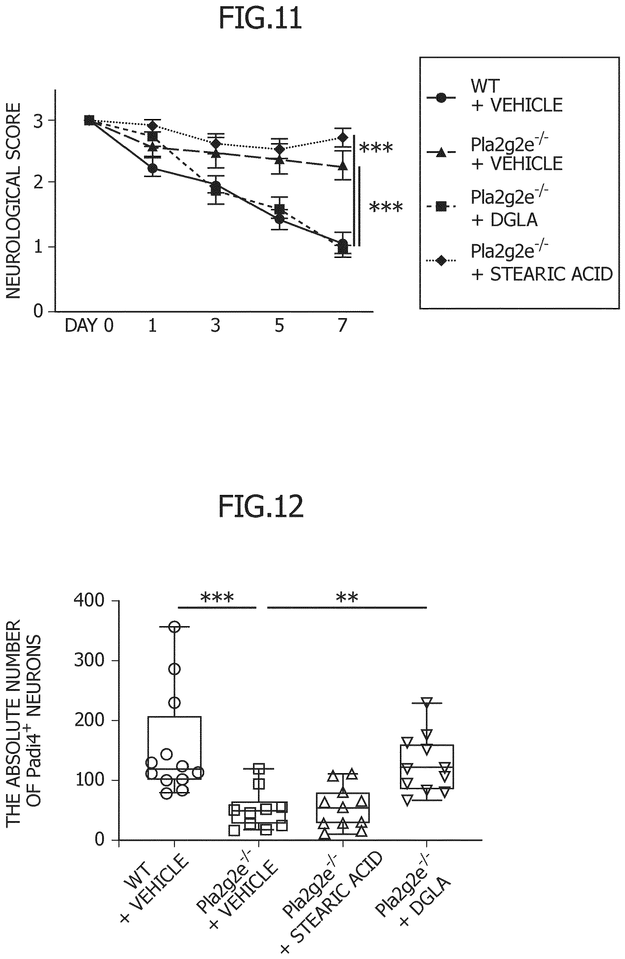

In addition, neurological symptoms after ischemia-reperfusion were evaluated over time according to the same procedure as in 2 above. The results are shown in Fig. 11. The improvement in neurological symptoms in the group treated with DGLA compared with the group treated with vehicle or stearic acid was confirmed (*** p < 0.001, two-way ANOVA with Tukey's post-test).

Also, sections were prepared from the cerebral tissue 4 days after ischemia-reperfusion and PADI4-positive neurons were detected by immunohistochemistry by the same procedure as in 2 above, and the number of cells per visual field (approximately 0.1 square millimeter) in the peri-infarct area was counted. The results are shown in Fig. 12. It was confirmed that the number of PADI4-positive neurons increased around the cerebral infarct area in the group treated with DGLA (** p < 0.01, *** p < 0.001, two-way ANOVA with Dunnett's post-test).

These results indicate that administration of DGLA and metabolites thereof can induce PADI4 expression, thereby protecting or repairing the infarcted cerebral tissue.

### 7. Analysis for cerebral infarction model of neuron-specific Padi4 conditional knockout mice

Neuron-specific Padi4-conditional knockout (cKO) mice (C57/B6J background) were generated by crossing Padi4 floxed mice (Pad4 flox: JAX No. 026708) and neuron-specific Cre-expressing mice (Vglut1-IRES2-Cre-D: JAX No. 023527; and Vgat-ires-cre: JAX No. 016962). As a control, myeloid cell-specific Padi4cKO mice (C57/B6J background) were generated by crossing Padi4 floxed mice and myeloid cell-specific Cre-expressing mice (LysMcre: JAX No. 004781). Cerebral infarction models of neuron-specific Padi4cKO mice, myeloid cell-specific Padi4cKO mice, and Padi4 floxed mice were generated (n=10, respectively) by the same procedure as in 1 above, and neurological symptoms over time after ischemia-reperfusion were evaluated by the same procedure as in 2 above.

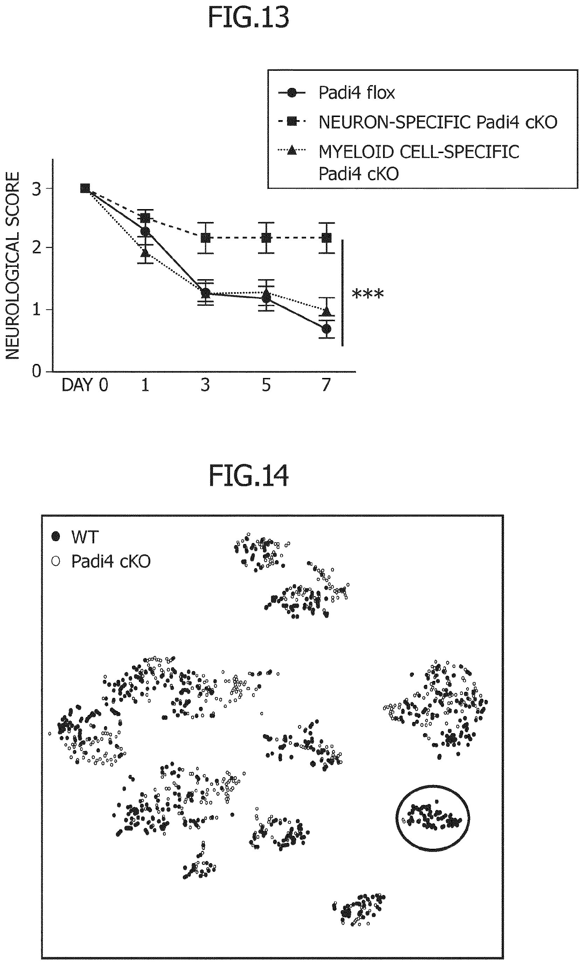

The results are shown in Fig. 13. The cerebral infarction model of neuron-specific Padi4cKO mice showed significantly worse neurological symptoms compared to the cerebral infarction models of myeloid cell-specific Padi4cKO mice and Padi4 floxed mice (*** p < 0.001, two-way ANOVA with Tukey's post-test).

Cerebral infarction models of neuron-specific Padi4cKO mice and wild-type mice were prepared by the same procedure as in 1 above. At 4 days after ischemia-reperfusion, the brain was isolated after transcardial perfusion with artificial cerebrospinal fluid. The brain was sliced into 300 µm thick sections and homogenized after treated with pronase. Neurons around the infarct area were isolated using anti-Thy1.2 Antibody (BioLegend) by a cell sorter (BD FACSAria III). Single-cell RNA-seq analysis was performed using Chromium Next GEM Single Cell 3' Kit (10x Genomics) and DNB-seq (MGI Tech), and the results of cell population clustering were visualized as a UMAP plot using Loupe Browser (10x Genomics). A gene ontology (GO) analysis was also performed for each cluster using g:profiler (https: /biit.cs.ut.ee/gprofiler/gost).

The results are shown in Fig. 14. The single-cell RNA-seq analysis classified neurons into 14 clusters, and the effects of ischemic stress were observed in all clusters compared to neurons derived from the normal brain. In particular, while neurons classified in cluster 4 (Fig. 14, circled cell group) were found around the infarct area of the cerebral infarction model of wild-type mice, they were not observed in the normal cerebral tissue or around the infarct area of the cerebral infarction model of neuron-specific Padi4cKO mice. Also, GO analysis also revealed that neurons classified in cluster 4 express a group of genes related to the formation of neural tissue, neurite outgrowth, and synapse formation (Table 2). Immunohistochemistry also confirmed co-staining of neurotrophic factor and PADI4, characteristic of cluster 4 (data not shown).

Table 2. GO terms characteristic of cluster 4

**[Table 2]**

| GO term | p-value |
|---|---|
| Synaptic signaling | 1.4×10⁻¹⁴ |
| Nervous system development | 2.5×10⁻¹⁴ |
| Neurogenesis | 1.5×10⁻¹³ |
| Generation of neurons | 1.6×10⁻¹¹ |
| Neuron differentiation | 6.6×10⁻¹¹ |

In addition, RNA-seq analysis was performed for gene expression of the entire neurons around the infarct area using the Ovation RNA-seq System (TECAN Genomics) and HiSeq (Illumina). The results also showed that the cerebral infarction model of neuron-specific Padi4cKO mice had a decreased expression of a group of genes associated with the formation of neural tissue, neurite outgrowth, and synapse formation (data not shown).

### 8. Therapeutic effect of 15-HETrE on cerebral infarction

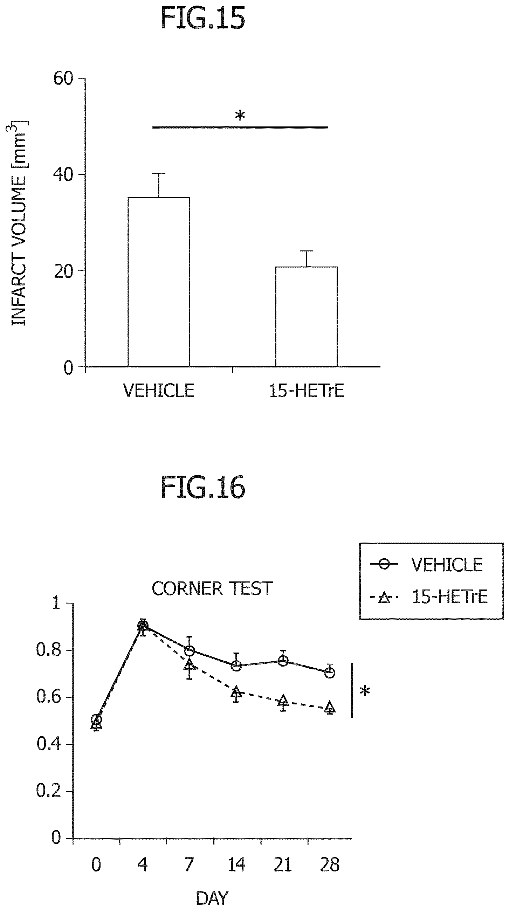

Cerebral infarction models of wild-type mice were prepared (n = 10) by the same procedure as in 1 above. At 24, 48, and 72 hours after ischemia-reperfusion, 15-HETrE (600 ng)/phosphate buffer was administered intravenously through the orbital venous plexus. The cerebral infarct volume 28 days after ischemia-reperfusion was calculated by the procedure in 6 above. The results are shown in Fig. 15. Administration of 15-HETrE decreased the cerebral infarct volume (*: p < 0.05 vs. vehicle (ethanol/phosphate buffer) treated mice (Student's t-test)).

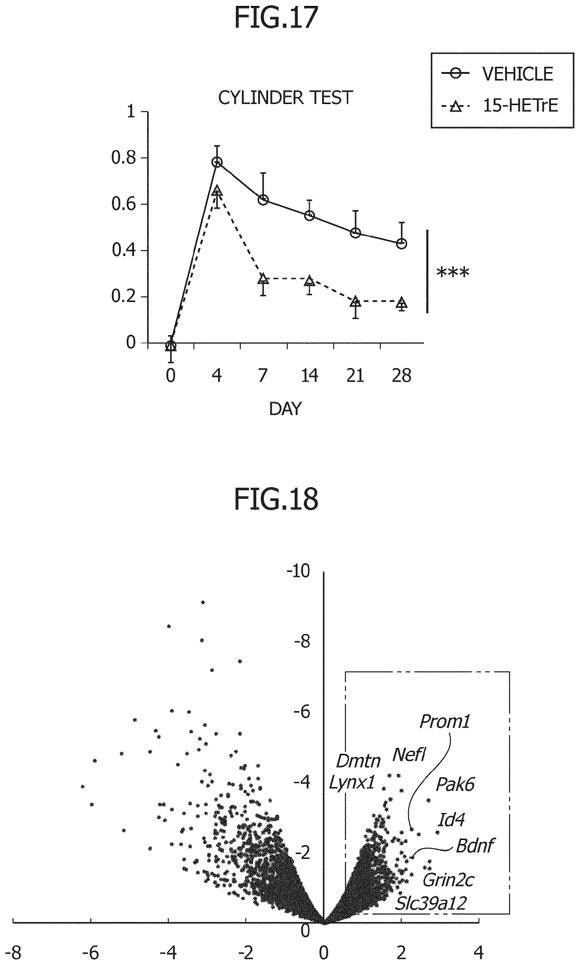

In addition, long-term neurological symptoms were evaluated over time up to 28 days after ischemia-reperfusion by a corner test and a cylinder test (Nat. Med., 2017 Jun; 23(6):723-732). The results of the corner test are shown in Fig. 16. The numbers on the vertical axis indicate (the number of turns in one direction at a corner out of 10 measurements)/10. The results of the cylinder test are shown in Fig. 17. The numbers on the vertical axis indicate (the number of contacts by the right upper limb (healthy side) with the cylinder wall - the number of contacts by the left upper limb) / (the number of contacts by the right upper limb + the number of contacts by the left upper limb + the number of simultaneous contacts by both upper limbs). These results indicate that administration of 15-HETrE improved neurological symptoms up to 28 days after the onset of cerebral infarction.

### 9. Induction of PADI4 expression by 15-HETrE

Cerebral infarction models of wild-type mice was prepared by the same procedure as in 1 above. At 24, 48, and 72 hours after ischemia-reperfusion, 15-HETrE (600 ng)/phosphate buffer was administered intravenously through the orbital venous plexus. Neurons around the infarct area were isolated from mice on day 4 after ischemia-reperfusion by the procedure in 7 above, and RNA-seq analysis was performed. The analysis results were visualized as a volcano plot using Loupe Browser, and GO analysis was performed using g:profiler.

The results are shown in Fig. 18. The vertical axis indicates p-value (-log10) and the horizontal axis indicates fold-change (log2). GO analysis of the group of genes for which expression increased (Fig. 18, framed group of genes) in the 15-HETrE-treated group revealed that a group of genes associated with neural tissue formation, neurite formation, and synapse formation were highly expressed in the 15-HETrE-treated group (Table 3).

### Table 3. GO terms characteristic of 15-HETrE-treated group

**[Table 3]**

| GO term | p-value |
|---|---|
| Nervous system development | 1.8×10⁻⁶⁴ |
| Synaptic signaling | 1.1×10⁻⁵⁸ |
| Neuron differentiation | 5.3×10⁻⁴⁷ |
| Neuron Projection development | 7.6×10⁻⁴¹ |
| Synapse organization | 2.4×10⁻³⁷ |

These results indicate that administration of DGLA and DGLA metabolites such as 15-HETrE can induce PADI4 expression, thereby repairing the infarcted cerebral tissue and restoring neurological functions.

## Claims

1. A composition for preventing or treating cerebrovascular disease in a subject, comprising an activator of peptidylarginine deiminase 4.

2. The composition according to claim 1, wherein the activator of peptidylarginine deiminase 4 is a dihomo-γ-linolenic acid metabolite.

3. The composition according to claim 2, wherein the dihomo-γ-linolenic acid metabolite is 15-hydroxyeicosatrienoic acid or a metabolite thereof.

4. The composition according to any one of claims 1-3, wherein the cerebrovascular disease is selected from the group consisting of cerebral hemorrhage, subarachnoid hemorrhage, cerebral infarction, and cerebrovascular dementia.

5. The composition according to any one of claims 1-4, wherein the composition is a pharmaceutical agent.

6. The composition according to claim 5, wherein the composition is administered within 48 hours of the onset of cerebrovascular disease.

7. The composition according to any one of claims 1 to 4, wherein the composition is a food or a drink.

8. A method of preventing or treating cerebrovascular disease in a subject, comprising a step of administering an activator of peptidylarginine deiminase 4 to the subject.

9. The method according to claim 8, wherein the activator of peptidylarginine deiminase 4 is a dihomo-γ-linolenic acid metabolite.

10. The method according to claim 9, wherein the dihomo-γ-linolenic acid metabolite is 15-hydroxyeicosatrienoic acid or a metabolite thereof.

11. The method according to any one of claims 8-10, wherein the cerebrovascular disease is selected from the group consisting of cerebral hemorrhage, subarachnoid hemorrhage, cerebral infarction, and cerebrovascular dementia.

12. The method according to any one of claims 8-11, wherein the step of administering is performed within 48 hours of the onset of cerebrovascular disease.
